# EUROPEAN PATENT APPLICATION

(11) **EP 4 778 580 A1**
(43) Date of publication of application: **22.07.2026**
(21) Application number: 25152810.5
(22) Date of filing: 20.01.2025
(51) Int. Cl.: A61P 19/02, A61K 39/00, A61K 38/17, C07K 14/78, A61P 37/02, C07K 14/74, A61P 29/00

(54) **MODIFIED COLLAGEN PEPTIDES**

(71) Applicant: Fraunhofer-Gesellschaft zur Förderung der angewandten Forschung e.V., 80686 München (DE)
(72) Inventor: ROMERO CASTILLO, Laura, 17165 Solna (SE); LINUSSON JONSSON, Anna, 90187 Umeå (SE); HOLMDAHL, Rikard, 17165 Solna (SE); KIHLBERG, Jan, 75123 Uppsala (SE)
(74) Representative: Kuttenkeuler, David

(57) **Abstract**

The invention describes modified collagen COL2₂₅₉₋₂₇₃ peptides and derived protein complexes thereof. The modified peptides of the invention are useful in the treatment or prevention of chronic inflammatory disorders such as rheumatoid arthritis (RA). The peptides, complexes comprising them and methods for their production are described. Further described are pharmaceutical compositions comprising the invented peptides and their uses in medicine.

## Description

### FIELD OF THE INVENTION

The invention provides modified collagen COL2₂₅₉₋₂₇₃ peptides and derived protein complexes thereof. The modified peptides of the invention are useful in the treatment or prevention of chronic inflammatory disorders such as rheumatoid arthritis (RA). Provided are the peptides, complexes comprising them and methods for their production. Further provided are pharmaceutical compositions comprising the inventive peptides and their uses in medicine.

### DESCRIPTION

In autoimmune diseases the patient's immune system recognize and attack the body-own ("self'). In consequence, antibodies and/or T cell responses are generated that recognize "self'-determinants" e.g. proteins, carbohydrates, nucleic acids and form immune complexes which activate the immune system because the "self-structures are recognized as foreign. This chronic condition can persist for years leading in the end to severe tissue and organ damage and possibly even to the death of the patient. There are many different autoimmune disorders which affect the body in various ways. For example, the brain is affected in individuals with multiple sclerosis, the gut is affected in individuals having Crohn's disease, and the synovium, bone and cartilage of various joints are affected in individuals suffering from rheumatoid arthritis. As autoimmune disorders progress destruction of one or more types of body tissues, abnormal growth of an organ, or changes in organ function may result. The autoimmune disorder may affect a single organ or tissue type or may affect multiple organs and tissues. Organs and tissues commonly affected by autoimmune disorders include red blood cells, blood vessels, connective tissues, endocrine glands (e.g., the thyroid or pancreas), muscles, joints, and the skin.

Examples of inflammatory and/ or autoimmune disorders include, but are not limited to, primary immune thrombocytopenia (ITP), systemic lupus erythematosus (SLE), rheumatoid arthritis (RA), autoimmune haemolytic anaemia (AIHA), type I diabetes, pemphigus vulgaris, Hashimoto's thyroiditis, autoimmune inner ear disease myasthenia gravis, pernicious anemia, Addison's disease, dermatomyositis, Sjogren's syndrome, dermatomyositis, multiple sclerosis, Reiter's syndrome, Graves disease, autoimmune hepatitis, familial adenomatous polyposis and ulcerative colitis.

New approaches are needed for the very early stages after clinical onset, but disease prevention is the ultimate goal. To improve treatment, the antigen-specific autoimmunity regulating the onset of joint inflammation must be addressed. Years before the clinical onset, autoimmune responses are triggered against citrullinated proteins in genetically predisposed individuals. This includes antibodies specific for the citrulline side-chain on many protein s, including cartilage proteins like type II collagen (COL2). Around the clinical onset, a more specific immune response to joint proteins, including the major joint protein, COL2, and to citrullinated COL2, appears. COL2 is of particular interest, as it is the major structural protein in cartilage as well as an autoantigenic component expressed in central lymphoid organs, the thymus and bone marrow. In physiology autoimmune cell activation to COL2 is protective but in arthritis an aberrant activation of COL2 specific B and T cells often occurs. Indeed, immunization of COL2 together with potent adjuvant induces the development of autoimmune arthritis in rodents (collagen-induced arthritis [CIA] model). Experimental arthritis is dependent on major histocompatibility complex region class II (MHCII) interaction; in the mouse, CIA is associated with the MHCII allele A^{q} and is strictly dependent on T-cell recognition of the galactosylated 259-273 COL2 epitope. Interestingly, T cells specific for the COL2₂₅₉₋₂₇₃ peptide occur in RA patients carrying the DRB1*0401 allele. COL2-reactive T cells are detectable in both healthy individuals and in RA patients and they predominantly recognize the immunodominant COL2₂₅₉₋₂₇₃ peptide interacting with the lysine's at position 264 and/or 270, these lysines can be recognized in different natural posttranslational forms like hydroxylation, galactosylation and galactoglucosylation. The form most recognized is galactosylation of lysine at position 264, forming the galCOL2 peptide which is an immunodominant target when bound to A^{q} (in the mouse) or DR (for example DRA/DRB1*0401) in humans. The function of these COL2-reactive T cells is not clear, as these cells could have regulatory or pathogenic functions. However, humanized mice expressing human DRB1*0401 develop CIA mediated by the COL2₂₅₉₋₂₇₃-specificT cells.

WO 2021/028347 discloses DR/COL2 peptide complexes comprising a chondroitin-binding peptide sequence at the C-terminal end of the polypeptide comprising the DR alpha chain and/or the DR beta chain, wherein the COL2 peptide is fused to the N -terminus of the DR alpha chain or the DR beta chain by a linker peptide. The complexes are for use in treating chronic inflammatory disease, such as arthritis, in human patients.

International patent publication WO 2021/028350 shows in situ glycosylated MHCII/COL2 peptide complexes, i.e., complexes naturally glycosylated during recombinant protein expression in the host cell. WO 2021/028350 also relates to methods of producing glycosylated MHCII/COL2 peptide complexes in mammalian cells as well as the use of such post-translationally modified, preferably glycosylated MHCII/COL2 complexed in treating rheumatoid arthritis (RA).

A variety of receptors acting in concert with the T cell receptor (co-receptors, which could be both inhibitory and stimulatory) have been characterized to be upregulated on natural as well as induced regulatory T cells. These include i.e. CTLA-4, PD-i, LAG-3, TIM-3, and TIGIT. The immunoregulatory function of these molecules have been characterized and their expression on CD4+ T cells is commonly used as markers of a regulatory T cell phenotype. These markers and additional molecules e.g. the transcription factor FOXP3, FR4 (folate receptor 4) and CD49b (a2 integrin chain), CD73 (ecto-5'-nucleotidase) and the secretion of interleukin-10 (review in 2) were tested in the development of a vaccine approach as indicators of the pharmacodynamic effect on the CD4+ T cell population. The above-mentioned markers were proven suitable to characterize the lymphocyte phenotype induced by therapeutic vaccination of collagen-induced arthritis (CIA) with a murine homologue to the human MHCII/gaICOL2 peptide complex was suggested to be similar to a Type 1 regulatory (TR1) cell. However, the respective in vitro stimulation of patient derived PBMCs with the respective human vaccine did not consistently upregulate the entire set of TR1 markers.

WO 2024/115605 discloses V-type immunoglobulin domain-containing suppressor of T-cell activation (VISTA) protein or mRNA as a diagnostic marker for the selection of patients suffering from autoimmune disorders for therapy, and as a means for monitoring therapy success of autoimmune disorders.

The above-described strategies require mostly glycosylated collagen peptides, such as COL2 peptides which are galactosylated at lysine₂₆₄. However, such peptides are not easy to produce and are not preferred candidates for a clinical development. Further it is known that autoreactive T cells specific for non-glycosylated COL2 may be present and cause unintended immune reactions (Bäcklund, Carlsen et al; Proc Natl Acad Sci U S A 2002 Jul 23;99(15):9960-5. doi: 10.1073/pnas.132254199).

Thus, it is an object of the invention to improve available strategies to tackle chronic inflammatory disorders. In particular the invention sets out to provide an alternative solution to collagen peptides that are galactosylated at position 264.

### BRIEF DESCRIPTION OF THE INVENTION

Generally, and by way of brief description, the main aspects of the present invention can be described as follows:

In **the first aspect,** the invention pertains to a modified COL2 peptide or protein, wherein the modified COL2 peptide or protein comprises a modified COL2 amino acid sequence motif COL2₂₅₉₋₂₇₃; or a variant COL2₂₅₉₋₂₇₃ motif having at least 80% sequence identity to human COL2₂₅₉₋₂₇₃; wherein the modified COL2 amino acid sequence motif is characterized by a modification of the amino acid side chains at positions 263 and/or 265 of the motif compared to the (wild-type) human sequence motif COL2₂₅₉₋₂₇₃.

In **a second aspect,** the invention pertains a protein complex comprising:
(i) A modified COL2 peptide or protein of any one of claims 1 to 13,
(ii) an extracellular region of an DR alpha chain comprising at least an alpha 1 domain;
(iii) an extracellular region of an DR beta chain comprising at least a beta 1 domain; and
wherein the modified COL2 is optionally fused to the N-terminus of the DR alpha chain or the DR beta chain by a linker peptide, preferably to the DR beta chain.

In a **third aspect,** the invention pertains to a pharmaceutical composition comprising the modified peptide of the first aspect, and/or the protein complex of the second aspect.

In **a fourth aspect,** the invention pertains to the peptide, protein, protein complex and/or pharmaceutical composition of the aspects of the invention for use in the medicine, in particular for use in the treatment or prevention of a chronic inflammatory disease. In an alternative, the fourth aspect pertains to a method of treating or preventing a disease in a subject, wherein the method comprises a step of administering a therapeutically effective amount of the peptide, protein, protein complex and/or pharmaceutical composition of the aspects of the invention.

In **a fifth aspect,** the invention pertains to a method for producing a composition, the method comprising steps of (i) Providing a modified COL2 peptide or protein of the first aspect or a protein complex of the second aspect; and (ii) admixing the modified COL2 peptide or protein or protein complex with at least one carrier and/or excipient to obtain the composition.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The term "protein" is used interchangeably with "amino acid sequence" or "polypeptide" and refers to polymers of amino acids of any length. These terms also include proteins that are post-translationally modified through reactions that include, but are not limited to, glycosylation, acetylation, phosphorylation, glycation or protein processing. Modifications and changes, for example fusions to other proteins, amino acid sequence substitutions, deletions or insertions, can be made in the structure of a polypeptide while the molecule maintains its biological functional activity.

For example certain amino acid sequence substitutions can be made in a polypeptide or its underlying nucleic acid coding sequence and a protein can be obtained with the same properties.

The term "polypeptide" typically refers to a sequence of more than 20 amino acids and the term "peptide" means sequences with up to 20 amino acids in length. However, the terms may be used interchangeably. A protein may form multimers such as dimers, wherein the dimer may be a heterodimer or a homodimer. The MHCII/COL2 peptide complex according to the invention comprise an extracellular region of an MHCII alpha chain and an extracellular region of an MHCII beta chain, which typically form a heterodimer which forms the binding groove to harbor the COL2 peptide fused to the N-terminus of one of the chains. However, the person skilled in the art will understand that two proteins forming a heterodimer can also be generated as a fusion protein forming a single polypeptide chain with the domains linked to each other, optionally via a flexible linker, i.e. a single chain heterodimer.

A "fusion protein" is defined as a protein which contains the complete sequences or any parts of the sequences of two or more originally separate natural or modified proteins. Fusion proteins can be constructed by genetic engineering approaches using recombinant DNA techniques by fusing the two or more genes or cDNAs, or parts thereof, that originally encode the two or more originally separate natural or heterologous proteins, or parts thereof. This results in a fusion protein with functional properties derived from each of the original proteins. Thus, a peptide or protein is linked to another protein by a peptide bond or preferably a linker peptide.

The term "genomic DNA", or "genome" is used interchangeably and refers to the heritable genetic information of a host organism. The genomic DNA comprises the DNA of the nucleus (also referred to as chromosomal DNA) but also of other cellular organelles (e.g., mitochondria).

The term "gene" as used herein refers to a DNA locus of heritable genomic sequence which affects an organism's traits by being expressed as a functional product or by regulation of gene expression. Genes and polynucleotides may include introns and exons as in a genomic sequence, or just the coding sequences as comprised in a cDNA, such as an open reading frame (ORF), comprising a start codon (methionine codon) and a translation stop codon. Genes and polynucleotides can also include regions that regulate their expression, such as transcription initiation, translation and transcription termination. Thus, also included are regulatory elements such as a promoter.

The terms "nucleic acid", "nucleotide", and "polynucleotide" as used herein are used interchangeably and refer to a single or double- stranded polymer of deoxyribonucleotide bases or ribonucleotide bases read from the 5' to the 3' end and include double stranded DNA (dsDNA), single stranded DNA (ssDNA), single stranded RNA (ssRNA), double stranded RNA (dsRNA), genomic DNA, cDNA, cRNA, recombinant DNA or recombinant RNA and derivatives thereof, such as those containing modified backbones. Preferably, a polynucleotide, particularly to be stably integrated into the mammalian genome, is a DNA or cDNA. Polynucleotides according to the invention can be prepared in different ways (e.g. by chemical synthesis, by gene cloning etc.) and can take various forms (e.g. linear or branched, single or double stranded, or a hybrid thereof, primers, probes etc.). The term "nucleotide sequence" or "nucleic acid sequence" refers to both the sense and antisense strands of a nucleic acid as either individual single strands or in the duplex.

The term "recombinant polynucleotide" as used herein refers to a polynucleotide derived from a different cell, organism or a different species from the recipient, e.g., a CHO cell or a HEK 293 cell, and introduced into the recipient using recombinant techniques. In the context of the present invention the skilled person would understand that it refers to a DNA or cDNA. A recombinant polynucleotide may also be referred to as transgene or a heterologous polynucleotide. Thus, it may be a gene or an open reading frame (ORF) coding for a recombinant protein. In the context of mammalian cells, such as HEK 293 or CHO cells "recombinant polynucleotide" refers to a polynucleotide derived from a different cell or artificially synthesized. The term "recombinant" refers to molecules such as polypeptides or polynucleic acid molecules formed by laboratory method of genetic recombination, such as molecular cloning. Such methods bring together genetic material from multiple sources or create sequences that do not naturally exist. When used with reference to portions of a nucleic acid, "recombinant" also includes a polynucleotide comprising two or more sequences that are not found in the same relationship to each other in nature or a polypeptide encoded by said polynucleotide. Recombinant may therefore also refer to a polynucleotide sequence, such as a gene or transgene, or a portion thereof, derived from the same cell line, but being inserted into the genome in a location in which it is not typically found, or a gene introduced into a cell of an organism in which it is not typically found.

As used herein a "recombinant polynucleotide", "recombinant gene" or "recombinant sequences" can be introduced into a target cell or host cell directly or preferably by using an "expression vector", preferably a mammalian expression vector. Methods used to construct vectors are well known to the person skilled in the art. Vectors may include, but are not limited to, plasmid vectors, cosmids, artificial/mini-chromosomes (e.g. ACE), or viral vectors such as retrovirus, adenovirus, adeno-associated virus and herpes simplex virus. The eukaryotic expression vectors will typically contain also prokaryotic sequences that facilitate the propagation of the vector in bacteria such as an origin of replication and antibiotic resistance genes for selection in bacteria. A variety of eukaryotic expression vectors, containing a cloning site into which a polynucleotide can be operably linked, are well known in the art. Usually expression vectors also comprise an expression cassette encoding a selectable marker, allowing selection of host cells carrying said expression marker.

The term "cytokine" refers to small proteins, which are released by cells and act as intercellular mediators, for example influencing the behavior of the cells surrounding the secreting cell. Cytokines may be secreted by immune or other cells, such as T-cells, B-cells, NK cells and macrophages. Cytokines may be involved in intercellular signaling events, such as autocrine signaling, paracrine signaling and endocrine signaling. They may mediate a range of biological processes including, but not limited to immunity, inflammation, and hematopoiesis. Cytokines may be chemokines, interferons, interleukins, lymphokines or tumor necrosis factors.

The term "expression" as used herein refers to transcription and/or translation of a nucleic acid sequence within a host cell. The level of expression of a gene product of interest in a host cell may be determined on the basis of either the amount of corresponding RNA that is present in the cell, or the amount of the polypeptide encoded by the selected sequence. For example, RNA transcribed from a selected sequence can be quantified by Northern blot hybridization, ribonuclease RNA protection, in situ hybridization to cellular RNA or by PCR, such as qPCR. Proteins encoded by a selected sequence can be quantitated by various methods, e.g. by ELISA, by Western blotting, by radioimmunoassay, by immunoprecipitation, by assaying for the biological activity of the protein, by immunostaining of the protein followed by FACS analysis or by homogeneous time-resolved fluorescence (HTRF) assays. The level of expression of a non-coding RNA, such as a miRNA or shRNA may be quantified by PCR, such as qPCR.

The term "gene product" refers to both the RNA polynucleotide and polypeptide that is encoded by a gene or DNA polynucleotide.

The term "proteinogenic amino acid" as used herein refers to all amino acids that are incorporated biosynthetically into proteins during translation. The term "proteinogenic" means protein creating. In eukaryotes there are 21 genetically encoding amino acids, i.e., proteinogenic amino acids, the 20 of the standard genetic code and selenocysteine. The 20 amino acids of the standard genetic code are alanine, arginine, asparagine, aspartic acid, cysteine, glutamic acid, glutamine, glycine, histidine, isoleucine, leucine, lysine, methionine, phenylalanine, proline, serine, threonine, tryptophane, tyrosine and valine.

The term "post-translational modification" or "post-translationally modified" as used herein refers to a naturally occurring modification of a lysine residue in the CII peptide that may occur when produced in cells. The post-translational modification of a lysine residue may result in hydroxylysine (Hyl) or is O-glycosylated Hyl, such as galactosyl-hydroxylysine or glucosylgalactosyl-hydroxylysine, preferably galactosyl-hydroxylysine.

The term "domain" as used herein refers to a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. For example, the alpha 1 domain of the MHC II alpha chain and the beta 1 domain of the MHC II beta chain each are folded polypeptide domains together forming the peptide binding groove of the MHC II molecule.

In **the first aspect,** the invention pertains to a modified COL2 peptide or protein, wherein the modified COL2 peptide or protein comprises a modified COL2 amino acid sequence motif COL2₂₅₉₋₂₇₃; or a variant COL2₂₅₉₋₂₇₃ motif having at least 80% sequence identity to human COL2₂₅₉₋₂₇₃; wherein the modified COL2 amino acid sequence motif is characterized by a modification of the amino acid side chains at positions 263 and/or 265 of the motif compared to the (wild-type) human sequence motif COL2₂₅₉₋₂₇₃.

In preferred embodiments, the modified COL2 peptide or protein comprises a modification at position 263 and/or 265 of human COL2 (SEQ ID NO: 1).

In other preferred embodiments of the invention, the COL2₂₅₉₋₂₇₃ peptide of the invention comprises, or consists of, a sequence shown in any one of SEQ ID No: 1 to 8.

In other preferred embodiments of the invention, the COL2₂₅₉₋₂₇₃ variant motif peptide of the invention comprises, or consists of, a sequence shown in any one of SEQ ID No: 9 to 23.

The modified COL2 peptide or protein of the invention, wherein in a preferred embodiment the modification is an exchange of the amino acid side chain to another proteinogenic side chain or to a non-proteinogenic side chain.

In another preferred embodiment the modification is at position 263, wherein the modification is a chemical modification of the phenyl ring phenylalanine, such as a substitution of a H with OH, Met, MetO, or a halogen, such as F, Cl, Br, or I.

Further preferred is a modified COL2 peptide or protein, wherein the phenylring at position 263 is replaced with a pyridine, for example such that the nitrogen is placed in all five possible positions (excluding the connecting beta carbon of the amino acid).

In some embodiments, the modified COL2 peptide or protein of the invention is modified such that the Phenylalanine side chain at position COL2₂₆₃ is modified by removing the -CH2-group or by extending the side chain, for example by modification of the carbon chain to (-CH₂-)ₙ wherein n is a natural number of 0 to 5, preferably is 0, 1, 2, or 3.

In another embodiment, the modified COL2 amino acid sequence motif comprises a sequence shown in SEQ ID NO: 2 to 5.

The modified COL2 peptide or protein of the invention in some embodiments is not galactosylated at position COL2₂₆₄(lysine).

Other embodiments of the invention pertain to a modification which is a replacement of the amino acid side chain at positions 263 and/or 265 with one of substituents selected from group consisting of:

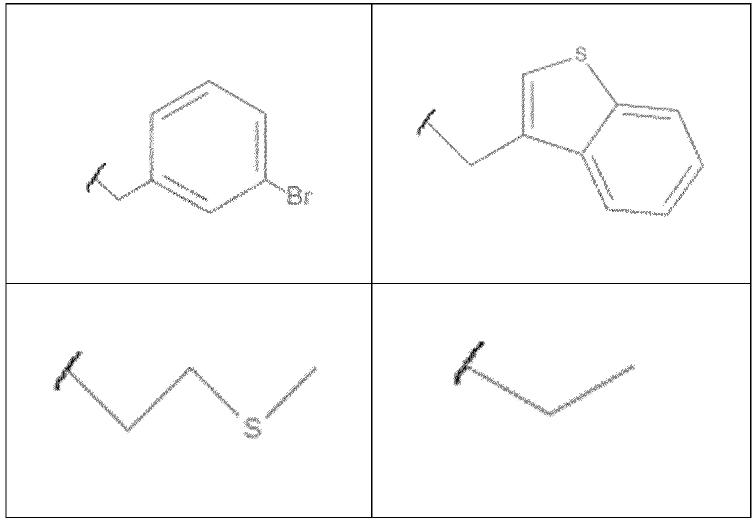

Preferred is a modified COL2 peptide or protein wherein the modification is a replacement of the amino acid side chain at position 263 with the chemical group:

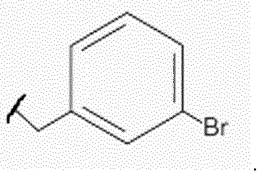

Preferred is a modified COL2 peptide or protein wherein the modification is a replacement of the amino acid side chain at position 263 with the chemical group:

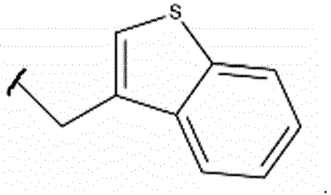

Preferred is a modified COL2 peptide or protein wherein the modification is a replacement of the amino acid side chain at position 265 with the chemical group:

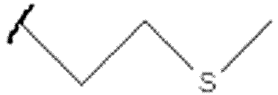

Preferred is a modified COL2 peptide or protein wherein the modification is a replacement of the amino acid side chain at position 265 with the chemical group:

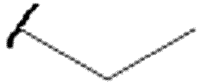

Further preferred modifications which may be combined with each other or with other modifications (and preferred embodiments thereof) as described above, are the following modifications:
- On position 264: hydroxylation, hydroxy-galactosylation, or hydroxy-galactoglucosylation;
- On position 270: hydroxylation, hydroxy-galactosylation, or hydroxy-galactoglucosylation;
- On position 266: substitution to GLN (via amidation of GLU);
- On position 272: substitution to GLN (via amidation of GLU);
- On position 269: substitution to GLU (via de-amidation of GLN);

The modified COL2 peptide of the invention in some embodiments may be based on, or comprise (or consist of) a sequence of a peptide variant as disclosed in WO 2021/028350. A selection of peptide variant sequences that could be used in context of the present invention are provided herein below.

The COL2 peptide comprises the amino acid sequence selected from the group consisting of AGFKGEQGPKG, AGFKGEQGPXG, AGFKGEXGPKG, AGFKGXQGPKG, AGFKXEQGPKG, AGFKGEXGPXG AGFKGXQGPXG and AGFKXEQGPXG. In certain embodiments the COL2 peptide comprises the amino acid sequence of AGFKGEQGPKG, AGFKGEQGPX₁G, AGFKGEX₂GPKG, AGFKGX₃QGPKG, AGFKX₄EQGPKG, AGFKGEX₂GPX₁G and AGFKGX₃QGPX1G, AGFKX₄EQGPX₁G, wherein X₁ is any of the proteinogenic amino acids except K, preferably R, A, G or Q, more preferably R; X₂ is any of the proteinogenic amino acids except Q; preferably A, R, H or G; X₃ is any of the proteinogenic amino acids except E, preferably A, D, Q or G; and X₄ is any of the proteinogenic amino acids except G, more preferably A, S, V or L. Preferably X₂, X₃ orX₄ is not K, more preferably X₁, X₂, X₃ orX₄ is not K. In certain embodiments the COL2 peptide comprises the amino acid sequence of AGFKGEQGPKG or AGFKGEQGPX₁G, preferably of AGFKGEQGPKGEP or AGFKGEQGPX₁GEP, more preferably of GIAGFKGEQGPKGEP or GIAGFKGEQGPX₁GEP. Preferably the COL2 peptide comprises the amino acid sequence of AGFKGEQGPKG (SEQ ID NO: 9) or AGFKGEQGPXG (SEQ ID NO: 10), preferably of AGFKGEQGPKGEP (SEQ ID NO: 18) or AGFKGEQGPXGEP (SEQ ID NO: 19), more preferably of GIAGFKGEQGPKGEP (SEQ ID NO: 21) or GIAGFKGEQGPXGEP (SEQ ID NO: 22). The COL2 peptide GIAGFKGEQGPKGEP corresponds to amino acids 259-273 of the triple helical type II collagen (COL2) region. COL2 peptides suitable for binding into the binding pocket are from 11 to 20 amino acids in length, preferably the COL2 peptide is 11 to 15 amino acids in length, more preferably the COL2 peptide is 13 to 15 amino acids in length. In one embodiment the COL2 peptide comprises the amino acid sequence AGFKGEQGPKG (SEQ ID NO: 9), more preferably AGFKGEQGPKGEP (SEQ ID NO: 18) and even more preferably of GIAGFKGEQGPKGEP (SEQ ID NO: 21). In one embodiment the second K (K270) may be mutated, preferably to R, A, G or Q, more preferably to R. Thus, also encompassed are embodiments, wherein the COL2 peptide comprises the amino acid sequence AGFKGEQGPXG (SEQ ID NO: 10), AGFKGEQGPXGEP (SEQ ID NO: 19) and GIAGFKGEQGPXGEP (SEQ ID NO: 22), wherein X may be any proteinogenic amino acid other than K, preferably X is R, A, G or Q, more preferably X is R. Thus, in one embodiment the COL2 peptide comprises the amino acid sequence AGFKGEQGPRG (SEQ ID NO: 17), AGFKGEQGPRGEP (SEQ ID NO: 20) and GIAGFKGEQGPRGEP (SEQ ID NO: 23). COL2 peptides encompassed by the present invention are disclosed in Table 2.

In **a second aspect,** the invention pertains to a protein complex comprising:
(iv) A modified COL2 peptide or protein of any one of claims 1 to 13,
(v) an extracellular region of an HLA-DR alpha chain comprising at least an alpha 1 domain;
(vi) an extracellular region of an HLA-DR beta chain comprising at least a beta 1 domain; and
wherein the modified COL2 is optionally fused to the N-terminus of the HLA-DR alpha chain or the HLA-DR beta chain by a linker peptide, preferably to the HLA-DR beta chain.

In a preferred embodiment of the invention the protein complex preferably comprises a chondroitin-binding peptide at the C- terminal end of the polypeptide comprising the HLA-DR alpha chain and/or the HLA-DR beta chain.

In a further embodiment, the protein complex of the invention preferably one of the following:
(a) the chondroitin-binding peptide is in its free form;
(b) the protein complex is not multimerized via the chondroitin-binding peptide; and/or
(c) the protein complex is not bound to a further molecule via the chondroitin-binding peptide.

In a further embodiment, the protein complex of the invention preferably one of the following:
(a) the extracellular region of the HLA-DR alpha chain comprises an alpha 1 and an alpha 2 domain; and/or
(b) the extracellular region of the HLA-DR beta chain comprises a beta 1 and a beta 2 domain.

The term "MHCII/COL2 peptide complex" as used herein refers to a soluble complex comprising the extracellular domains of an MHCII protein or part thereof forming the peptide binding groove and a COL2 peptide (CM peptide), wherein the peptide is fused (i.e., linked via a linker peptide) to the N-terminus of either the alpha or the beta chain. Preferably the CM peptide is linked to the N-terminus of the MHCII beta chain. An MHCII protein comprises an alpha 1 domain and an alpha 2 domain, which form the extracellular domain of the alpha chain and a beta 1 domain and a beta 2 domain, which form the extracellular domain of the beta chain. The term "extracellular domain" and "extracellular region" are used synonymously herein. The alpha 1 domain and the beta 1 domain form the peptide binding groove, i.e., the site that interacts and binds the peptide, such as the CM peptide. Thus, the MHCII/COL2 peptide complex comprises at least the alpha 1 domain and the beta 1 domain of the MHCII protein. Preferably the MHCII/COL2 peptide complex comprises the alpha 1 domain, the alpha 2 domain, the beta 1 domain and the beta 2 domain of the MHCII protein. MHCII proteins are a class of major histocompatibility complex (MHC) region expressed cell membrane proteins normally found only on antigen-presenting cells (APCs) such as dendritic cells, mononuclear phagocytes, such as monocytes and macrophages, and B cells but to some degree also on T cells, neutrophil, endothelial cells and fibroblasts. The antigens presented by MHCII molecules are derived from extracellular proteins, while MHCI molecules present cytosolic or intracellular peptides. Extracellular proteins are endocytosed, digested and loaded onto MHCII proteins forming an MHCII/peptide complex. The loaded complex is then transferred to the cell surface, where it is presented to effector cells. In humans, the MHC proteins are referred to as human leukocyte antigens (HLA). Thus, as used herein MHCII protein encompasses HLA class II proteins. HLAs corresponding to MHCII proteins are DP, DM, DOA, COB, DQ and DR. For RA there is a genetic association with certain alleles of the DRB1 locus, encoding DR beta chain type 1, coding for an amino acid consensus motif (Q/R R/K R A A) on the beta-chain of the peptide binding pocket of the MHCII molecule DR (amino acid position 70-74, the so called "shared epitope"). Examples for RA associates DRB1 alleles are QKRAA-coding alleles DRB1* 0401 and 0409, QRRAA-coding alleles: DRB1* 0404, 0405, 0408, 0101 , 0102 and 1402, RRRAA-coding allele: DRB1* 1001 and DKRAA-coding allele: DRB1* 1303. In one embodiment, the extracellular region of the MHCII alpha chain and the extracellular region of the MHCII beta chain are therefore derived from DR, preferably at least the alpha 1 domain is from DRA*0101 and at least the beta 1 domain is from a DR allele selected from the group consisting of DRB1*0401 , DRB1*0404 and DRB1*0405, DRB1*0408, DRB1*0409, DRB1*0101 , DRB1*0102, DRB1*1001 , DRB1*1402 and DRB1*1303, preferably DRB1*0401 , DRB1*0404, DRB1*0405, DRB1*0408, DRB1*0409, DRB1*0101, DRB1*0102, DRB1*1001 and DRB1*1402, more preferably DRB1*0401 , DRB1*0404, DRB1*0101 and DRB1*0405, even more preferably DRB1*0401. More preferably the alpha 1 domain and the alpha 2 domain is from DRA*0101 (alpha 1 and 2 domain: amino acids 19-200 of SEQ ID NO: 24) and the beta 1 domain and the beta 2 domain is from a DR allele selected from the group consisting of DRB1*0401 , DRB1*0404 and DRB1*0405, DRB1*0408, DRB1*0409, DRB1*0101 , DRB1*0102, DRB1*1001, DRB1*1402 and DRB1*1303, preferably DRB1*0401 , DRB1*0404, DRB1*0405, DRB1*0408, DRB1*0409, DRB1*0101 , DRB1*0102, DRB1*1001 and DRB1*1402, more preferably DRB1*0401 , DRB1*0404, DRB1*0101 and DRB1*040, even more preferably DRB1*0401 (beta 1 and 2 domain: amino acids 60-250 of SEQ ID NO: 25). In mice collagen-induced arthritis (CIA) is associated with the mouse MHCII Aq allele (A^{q}).

In one preferred embodiment the complex is preferred, wherein at least the alpha 1 domain is from DRA*0101 and at least the beta 1 domain is from a DR allele selected from the group consisting of DRB1*0401 , DRB1*0404, DRB1*0405, DRB1*0408, DRB1*0409, DRB1*0101 , DRB1*0102, DRB1*1001 , DRB1*1402 and DRB1*1303, preferably DRB1*0401.

In context of the invention the chondroitin binding peptide may comprise 5 to 20 amino acids, preferably 6 to 12 amino acids.

In other embodiments, the chondroitin binding peptide is a polyhistidine tag, preferably at least a hexahistidine tag.

The protein complex of the invention may be further preferred wherein
(a) the extracellular region of the DR alpha chain comprising at least an alpha 1 domain; and
(b) the extracellular region of the DR beta chain comprising at least a beta 1 domain are expressed as a single fusion polypeptide; and optionally
(c) the COL2 peptide is fused to the N-terminus of the DR alpha chain or the DR beta chain by a linker peptide, preferably to the DR beta chain.

Yet another embodiment of the second aspect relates to a protein complex of the invention, comprising
(a) a first polypeptide comprising the extracellular region of the DR alpha chain comprising at least an alpha 1 domain;
(b) a second polypeptide comprising the extracellular region of the DR beta chain comprising at least a beta 1 domain;
(c) the COL2 peptide, optionally fused to the N-terminus of the DR alpha chain or the DR beta chain by a linker peptide, preferably to the DR beta chain; and
(d) wherein the DR alpha chain is fused at its C-terminal end to a first functional domain of a leucine zipper heterodimerisation motif and the DR beta chain is fused at its C-terminal end to a second complementary functional domain of a leucine zipper heterodimerisation motif.

Further preferred is that the first functional domain and the second complementary functional domain of the complex of the invention are
(a) an acidic and a basic leucine zipper heterodimerisation domain; and/or
(b) a jun-fos leucine zipper motif.

In a **third aspect,** the invention pertains to a pharmaceutical composition comprising the modified peptide of the first aspect, and/or the protein complex of the second aspect.

A preferred pharmaceutical composition of the invention is a vaccine composition.

In **a fourth aspect,** the invention pertains to the peptide, protein, protein complex and/or pharmaceutical composition of the aspects of the invention for use in the medicine, in particular for use in the treatment or prevention of a chronic inflammatory disease. In an alternative, the fourth aspect pertains to a method of treating or preventing a disease in a subject, wherein the method comprises a step of administering a therapeutically effective amount of the peptide, protein, protein complex and/or pharmaceutical composition of the aspects of the invention.

In context of the invention the chronic inflammatory disease is rheumatoid arthritis, osteoarthritis, psoriatic arthritis, non- radiographic axial spondyloarthritis, ankylosing spondylitis, juvenile idiopathic arthritis, relapsing polychondritis, systemic lupus erythematosus, Lyme disease, Meniere diseases, autoimmune inner ear disease (AIED), or Still's disease.

A disease preferably targeted for treatment or prevention in context of the invention is rheumatoid arthritis.

In **a fifth aspect,** the invention pertains to a method for producing a composition, the method comprising steps of (i) Providing a modified COL2 peptide or protein of the first aspect or a protein complex of the second aspect; and (ii) admixing the modified COL2 peptide or protein or protein complex with at least one carrier and/or excipient to obtain the composition.

The method in a preferred embodiment comprises a subsequent step to step (i-1) comprising testing immunogenicity of modified COL2 peptide or protein; and/or comprising testing; and (i-2) comprising continuing with step (ii) if the modified COL2 peptide or protein is immunogenic.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

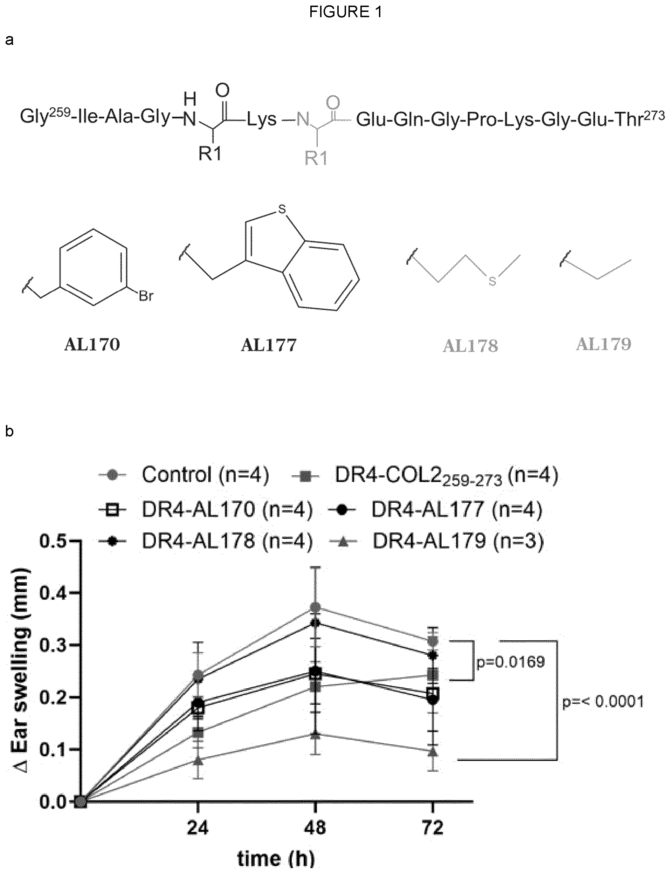

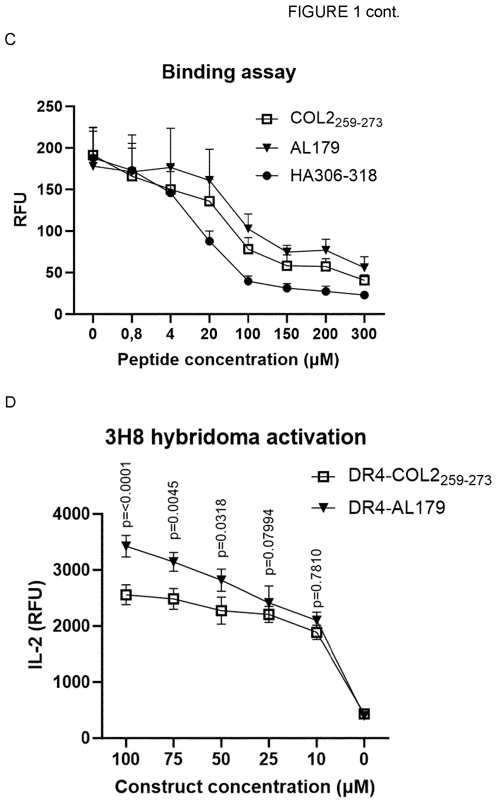

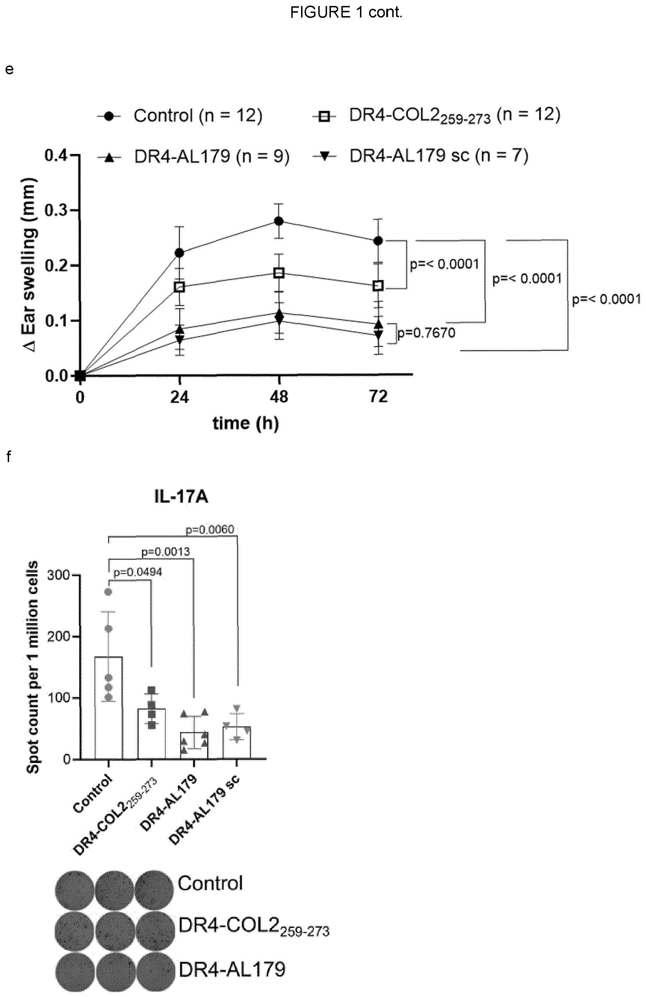

**Figure 1****:** (a) Schematic design of the synthesized COL2-peptides. (b) DTH reactions (ear swelling) following treatment with DR4-COL2 complexes using osmotic pumps. Number of mice per group are indicated in parentheses. (c) Binding affinity of AL179 and COL2₂₅₉₋₂₇₃ with the DRB1*04:01 molecule. Influenza hemagglutinin peptide (HA306-318) was used as a positive control. Data is representative of three experiments. (d) Native-COL2 specific T hybridoma (3H8) activation by DR4-COL2 complexes. T cell activation was assessed by measurement of the IL-2 secreted into the medium by ELISA. Data is representative of three experiments. (e) DTH reactions (ear swelling) following treatment with DR4-COL2 complexes using osmotic pumps or subcutaneous injections. Number of mice per group are indicated in parentheses. (f) Measurement of antigen-specific IL-17A T cell response against the COL2 protein, assessed by ELISpot in inguinal lymph nodes 72 h after the DTH model. Results are expressed as mean ± SEM. Statistics were determined using a 2-way ANOVA with Sidak's multiple comparisons test. (Control: DR4-CLIP).

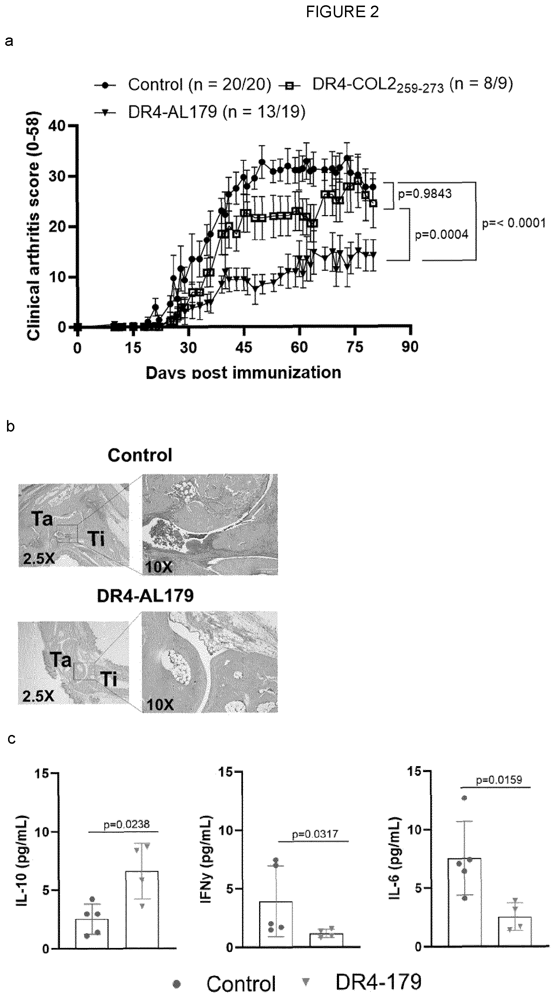

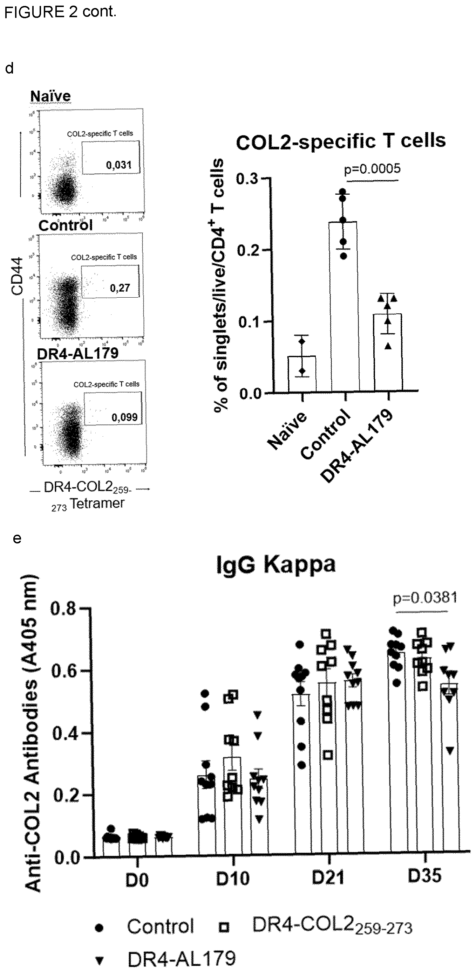

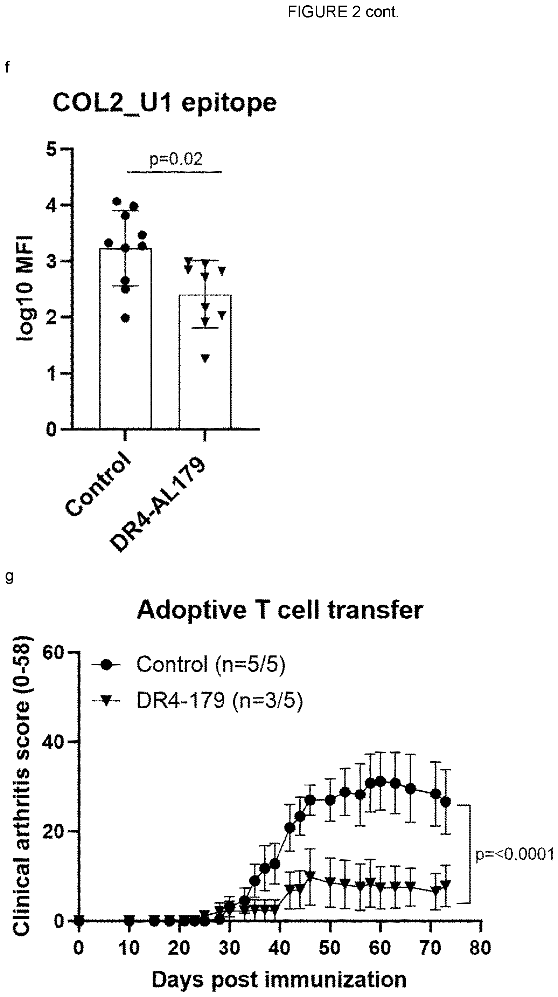

**Figure 2****:** shows (a) CIA in vaccinated mice as assessed by mean clinical score of arthritis severity and incidence of arthritis (Number of arthritic mice/total number of mice). DRB1*04:01-expressing mice (Parker) were immunized with 100 µg COL2 on day 0 and boosted on day 35 with 50 µg COL2. On day 4 and day 32, mice were vaccinated s.c. with DR4-peptide complexes using 3 s.c administrations. Data from two pooled experiments. (b) Representative histological examination by H&E staining of the tibia (Ti) and talus (Ta) from control and DR4-AL179 vaccinated mice. Cell infiltration and cartilage/bone destruction are demonstrated in the control group. (c) Serum cytokine levels of IL-10, IFN-γ and IL-6 after 20 DPI. (d) Vaccination with DR4-AL179 complexes reduces frequencies of COL2-specific T cells at the day 80 (terminal point). Parker mice were immunized with COL2/CFA, and 4 DPI were treated (100 µg) by 3 s.c injections. 15 DPI T cells in draining lymph nodes (dLN) were analyzed with DR4-COL2₂₅₉₋₂₇₃ tetramer staining using flow cytometry. Representative gates are shown. (e) Anti-COL2 antibody levels of total IgG (anti-kappa) from vaccinated mice at different time points along CIA model measured by ELISA. (f) IgG antibodies binding to the triple helical COL2 epitope U1 in sera collected from vaccinated mice at 45 DPI measured by bead-based flow immunoassay and quantified as Log10-transformed MFI. (g) Clinical scoring of arthritis severity following the transfer of T cells from DR4-CLIP-vaccinated and DR4-AL179-vaccinated Parker mice into COL2 immunized recipient Parker mice. The number of arthritic mice/total number of mice are represented. Results are expressed as mean ± SEM. Each dot represents an individual mouse. Statistics in (a, d, e and g) experiments represented were determined using 2-way ANOVA with Sidak's multiple comparisons test, and in c and f were determined with a two-tailed Mann-Whitney test. (DPI- Days post-immunization; s.c- Subcutaneous; Control: DR4-CLIP).

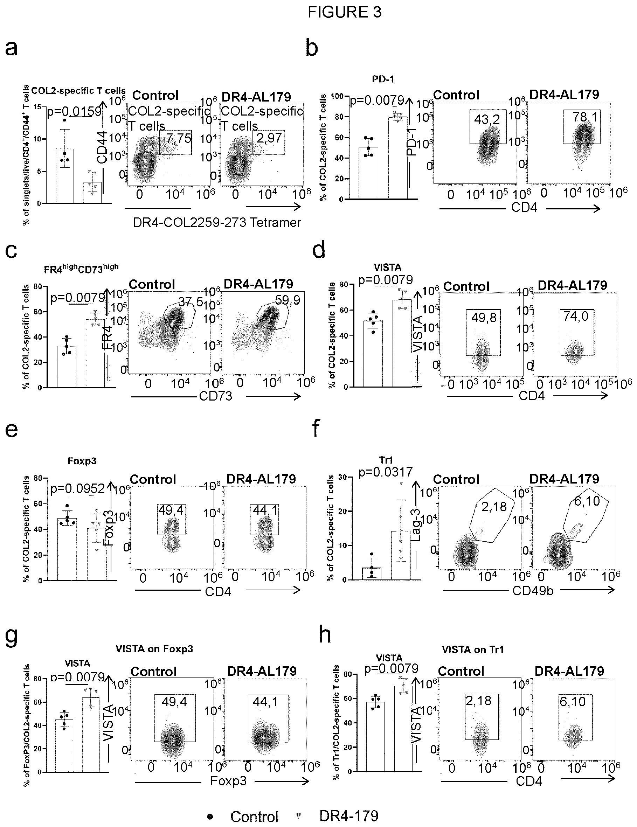

**Figure 3****:** shows (a) Vaccination with DR4-AL179 complexes reduces frequencies of COL2-specific T cells after 15 DPI. Parker mice were immunized with COL2/CFA, and at 4 DPI mice were vaccinated with DR4-peptide complexes by 3 s.c injections. 15 DPI splenocytes and dLN cells were recovered, stained with the PE-labeled DR4-COL2₂₅₉₋₂₇₃ tetramer and enriched using anti-PE magnetic beads for immunophenotyping by flow cytometry. Representative flow cytometry plots depicting the gating strategy for DR4-COL2₂₅₉₋₂₇₃+/CD44+ (COL2-specific) cells among live CD4+ T cells. (b-d) Vaccination with the DR4-AL179 complexes increases the frequency of PD-1, CD73, FR4, and VISTA among COL2-specific CD4+ T cells. Representative flow cytometry plots depicting the gating strategy for PD-1, CD73, FR4 and VISTA in DR4-COL2₂₅₉₋₂₇₃+/CD44+/CD4+ T cells. (e) Frequency of Foxp3+ among CD4+ in COL2-specific T cells. Representative flow cytometry plots depicting the gating strategy for Foxp3 in DR4-COL2₂₅₉₋₂₇₃+/CD44+/CD4+ T cells. (f) Frequency of Tr1 (LAG3+/CD49+) among CD4+ in COL2-specific T cells. Representative flow cytometry plots depicting the gating strategy for LAG3+/CD49+ (Tr1) in DR4-COL2₂₅₉₋₂₇₃+/CD44+/CD4+ T cells. (g)) Frequency of VISTA+ among FOXP3+ in COL2-specific T cells. Representative flow cytometry plots depicting the gating strategy for VISTA+ in FOXP3+/DR4-COL2₂₅₉₋₂₇₃+/CD44+/ CD4+ T cells. (e) Frequency of VISTA+ among Tr1 in COL2-specific T cells. Representative flow cytometry plots depicting the gating strategy for VISTA+ in LAG3+/CD49+/DR4-COL2₂₅₉₋₂₇₃+/CD44+/CD4+ T cells. Results are expressed as mean ± SEM. Each dot represents an individual mouse. Statistics were determined with a two-tailed Mann-Whitney test. (Control: DR4-CLIP).

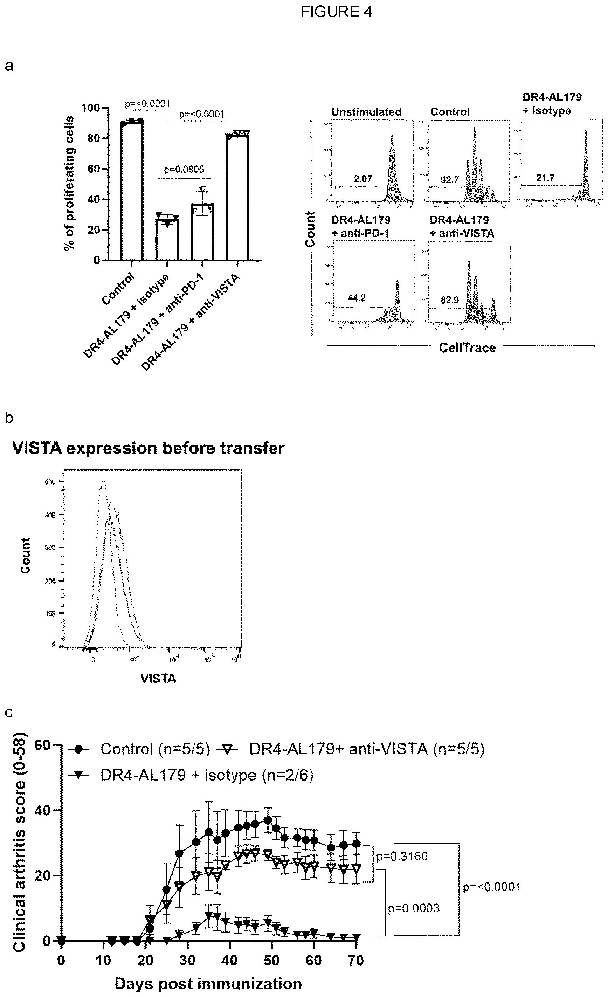

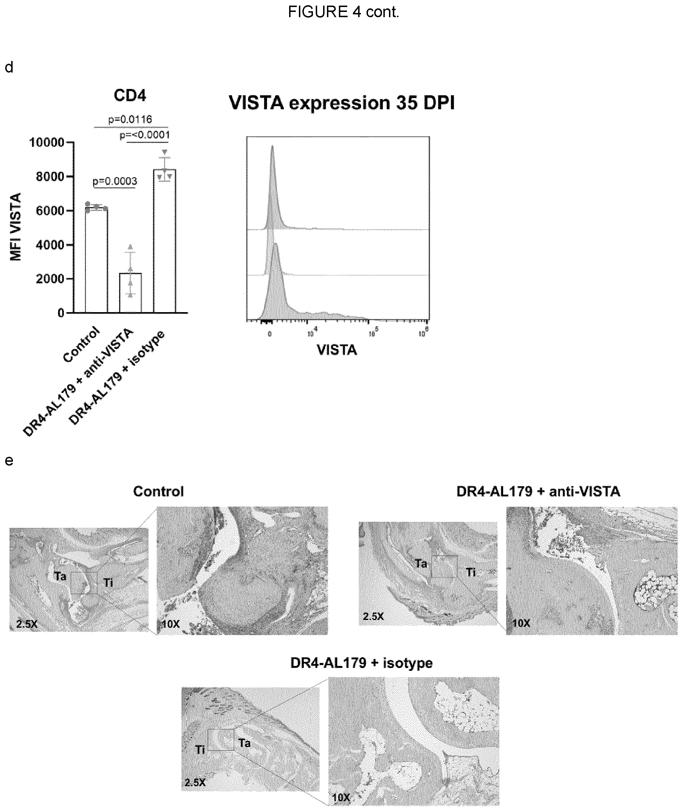

**Figure 4****:** shows (a) Parker mice were vaccinated with DR4-peptide complexes and 15 DPI splenocytes were recovered. CD4+T cells were purified and cocultured with purified violet cell trace-labeled CD4+ T cells from naïve Parker mice. CD4+T cells from DR4-AL179 vaccinated mice were incubated for 1 h at 4 °C with either 100 µg/mL anti-VISTA antibody, anti-PD-1 antibody or isotype control. Afterward, cells were washed and added to the naive Parker T cells. Flow cytometry was performed 3 d after stimulation with anti-CD3/CD28 antibodies. Quantification (Left) and representative gating (Right) are shown. (b-e) Adoptive transfer of CD4+ T cells from DR4-AL179 vaccinated mice transferred into COL2 immunized recipient Parker mice. A control group received T cells from DR4-CLIP vaccinated mice. CD4+ T cells from DR4-AL179 vaccinated mice were incubated for 1 h with either anti-VISTA (DR4-AL179 + anti-VISTA) or isotype control (DR4-AL179 + isotype) antibodies before being transferred. (b) The expression of VISTA within the different groups was assessed by flow cytometry before transfer. (c) Arthritis clinical score in recipient mice after T cell transfer. The number of arthritic mice/total number of mice are shown. (d) The expression of VISTA within the different groups was assessed by flow cytometry after 35 DPI in peripheral blood. (e) Representative histological examination by H&E staining of the tibia (Ti) and talus (Ta) assessment in recipient mice. Results are expressed as mean ± SEM. Each dot represents an individual mouse. Statistics were determined by using 2-way ANOVA with Sidak's multiple comparisons test. (Control: DR4-CLIP).

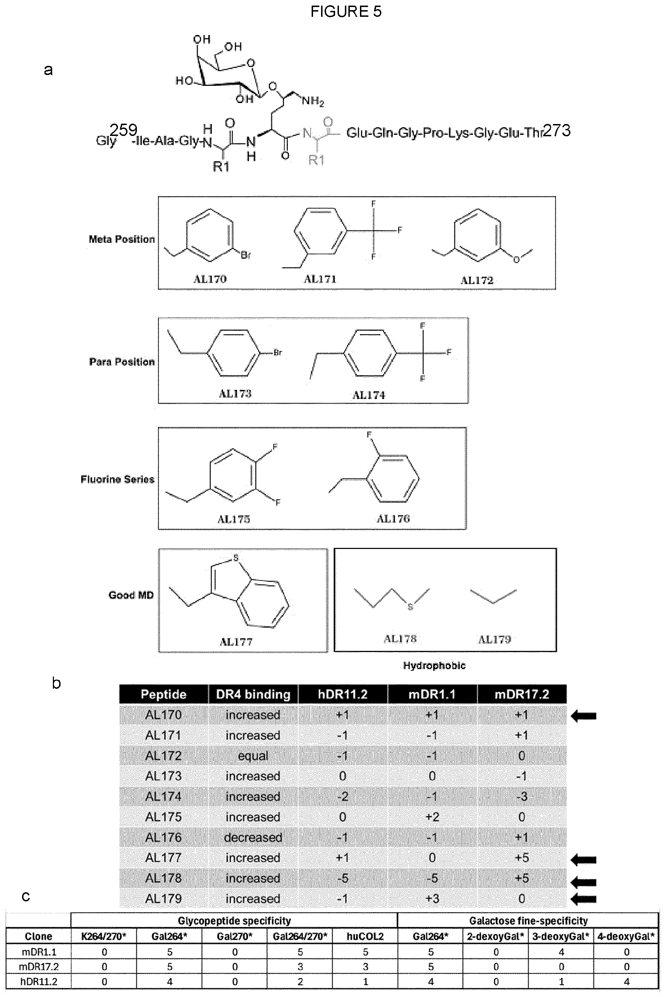

**Figure 5****:** shows (a) Schematic design of the synthesized COL2 glycopeptides. (b) Summary of the results for each glycopeptide based on the DRB1*04:01 (DR4) binding affinity and T cell hybridoma activation (c) The specificity of the different T cell hybridoma clones activated by the COL2 glycopeptides.

The sequences show:

**Table 1:**

| **SEQ ID NO:** | **Sequence** |
|---|---|
| 1 | GIAGFKGEQGPKGET |
| 2 | GIAGFKGEQGPKGQT |
| 3 | GIAGFKGQQGPKGET |
| 4 | GIAGFKGQQGPKGQT |
| 5 | GIAGFKGEEGPKGET |
| 6 | GIAGFKGEEGPKGQT |
| 7 | GIAGFKGEQGPKGET |
| 8 | GIAGFKGEQGPKGQT |

and

**Table 2:**

| **SEQ ID NO:** | **SEQUENCE:** |
|---|---|
| 9 | AGFKGEQGPKG |
| 10 | AGFKGEQGPX₁G* |
| 11 | AGFKGEX₂GPKG* |
| 12 | AGFKGX₃QGPKG* |
| 13 | AGFKX₄EQGPKG* |
| 14 | AGFKGEX₂GPX₁G* |
| 15 | AGFKGX₃QGPX1G* |
| 16 | AGFKX₄EQGPX₁G* |
| 17 | AGFKGEQGPRG |
| 18 | AGFKGEQGPKGEP* |
| 19 | AGFKGEQGPX₁GEP* |
| 20 | AGFKGEQGPRGEP |
| 21 | GIAGFKGEQGPKGEP |
| 22 | GIAGFKGEQGPX₁GEP |
| 23 | GIAGFKGEQGPRGEP |

*wherein X₁ is any of the proteinogenic amino acids except K, preferably R, A, G or Q, more preferably R; X₂ is any of the proteinogenic amino acids except Q; preferably A, R, H or G; X₃ is any of the proteinogenic amino acids except E, preferably A, D, Q or G; and X₄ is any of the proteinogenic amino acids except G, more preferably A, S, V or L.

SEQ ID NO: 24 shows the amino acid sequence of DR4 construct alpha-chain:

Met Lys Leu Cys Ile Leu Leu Ala Val Val Ala Phe Val Gly Leu Ser
1               5                   10                  15
Leu Gly Ile Lys Glu Glu His Val Ile Ile Gln Ala Glu Phe Tyr Leu
            20                  25                  30
Asn Pro Asp Gln Ser Gly Glu Phe Met Phe Asp Phe Asp Gly Asp Glu
        35                  40                  45
Ile Phe His Val Asp Met Ala Lys Lys Glu Thr Val Trp Arg Leu Glu
    50                  55                  60
Glu Phe Gly Arg Phe Ala Ser Phe Glu Ala Gln Gly Ala Leu Ala Asn
65                  70                  75                  80
Ile Ala Val Asp Lys Ala Asn Leu Glu Ile Met Thr Lys Arg Ser Asn
                85                  90                  95
Tyr Thr Pro Ile Thr Asn Val Pro Pro Glu Val Thr Val Leu Thr Asn
            100                 105                 110
Ser Pro Val Glu Leu Arg Glu Pro Asn Val Leu Ile Cys Phe Ile Asp
        115                 120                 125
Lys Phe Thr Pro Pro Val Val Asn Val Thr Trp Leu Arg Asn Gly Lys
    130                 135                 140
Pro Val Thr Thr Gly Val Ser Glu Thr Val Phe Leu Pro Arg Glu Asp
145                 150                 155                 160
His Leu Phe Arg Lys Phe His Tyr Leu Pro Phe Leu Pro Ser Thr Glu
                165                 170                 175
Asp Val Tyr Asp Cys Arg Val Glu His Trp Gly Leu Asp Glu Pro Leu
            180                 185                 190
Leu Lys His Trp Glu Phe Asp Ala Ser Gly Gly Gly Glu Asn Leu Tyr
        195                 200                 205

Phe Gln Gly Gly Gly Gly Ser Leu Thr Asp Thr Leu Gln Ala Glu Thr
    210                 215                 220
Asp Gln Leu Glu Asp Glu Lys Ser Ala Leu Gln Thr Glu Ile Ala Asn
225                 230                 235                 240
Leu Leu Lys Glu Lys Glu Lys Leu Glu Phe Ile Leu Ala Ala His Gly
                245                 250                 255
Gly Gly Gly Ser Gly Leu Asn Asp Ile Phe Glu Ala Gln Lys Ile Glu
            260                 265                 270
Trp His Glu
        275

SEQ ID NO: 25 shows the amino acid sequence of DR4 construct beta-chain with hCll259-273 peptide

Met Lys Leu Cys Ile Leu Leu Ala Val Val Ala Phe Val Gly Leu Ser
1               5                   10                  15
Leu Gly Ser Ala Trp Ser His Pro Gln Phe Glu Lys Gly Ile Ala Gly
            20                  25                  30
Phe Lys Gly Glu Gln Gly Pro Lys Gly Glu Pro Ser Gly Gly Gly Ser
        35                  40                  45
Leu Val Pro Arg Gly Ser Gly Gly Gly Ser Gly Asp Thr Arg Pro
    50                  55                  60
Arg Phe Leu Glu Gln Val Lys His Glu Cys His Phe Phe Asn Gly Thr
65                  70                  75                  80
Glu Arg Val Arg Phe Leu Asp Arg Tyr Phe Tyr His Gln Glu Glu Tyr
                85                  90                  95
Val Arg Phe Asp Ser Asp Val Gly Glu Tyr Arg Ala Val Thr Glu Leu
            100                 105                 110
Gly Arg Pro Asp Ala Glu Tyr Trp Asn Ser Gln Lys Asp Leu Leu Glu
        115                 120                 125
Gln Lys Arg Ala Ala Val Asp Thr Tyr Cys Arg His Asn Tyr Gly Val
    130                 135                 140
Gly Glu Ser Phe Thr Val Gln Arg Arg Val Tyr Pro Glu Val Thr Val
145                 150                 155                 160
Tyr Pro Ala Lys Thr Gln Pro Leu Gln His His Asn Leu Leu Val Cys
                165                 170                 175
Ser Val Asn Gly Phe Tyr Pro Gly Ser Ile Glu Val Arg Trp Phe Arg
            180                 185                 190
Asn Gly Gln Glu Glu Lys Thr Gly Val Val Ser Thr Gly Leu Ile Gln
        195                 200                 205
Asn Gly Asp Trp Thr Phe Gln Thr Leu Val Met Leu Glu Thr Val Pro
    210                 215                 220
Arg Ser Gly Glu Val Tyr Thr Cys Gln Val Glu His Pro Ser Leu Thr
225                 230                 235                 240
Ser Pro Leu Thr Val Glu Trp Arg Ala Arg Ser Gly Gly Gly Glu Asn
                245                 250                 255
Leu Tyr Phe Gln Gly Gly Gly Gly Ser Arg Ile Ala Arg Leu Glu Glu
            260                 265                 270
Lys Val Lys Thr Leu Lys Ala Gln Asn Ser Glu Leu Ala Ser Thr Ala
        275                 280                 285
Asn Met Leu Arg Glu Gln Val Ala Gln Leu Lys Gln Lys Val Met Asn
    290                 295                 300
His His His His His His His
305                 310

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Developing modified COL2-peptides as potential vaccine candidates for RA.

To develop improved vaccine candidates, the inventors conducted *in vitro* testing of a range of modified glycopeptides derived from the parent peptide COL2₂₅₉₋₂₇₃, galactosylated at lysine 264 (Fig. 5a). We assessed both MHCII binding and T cell activation using different T cell hybridoma clones specific for glycosylated epitopes. The specificity of the T cell hybridomas utilized is detailed in Fig. 5a. The results highlighted AL170, AL177, AL178 and AL179 peptides, which were selected based on their enhanced MHCII binding and alterations in T cell contact points (Fig. 5a).

Due to the presence in humans of autoreactive T cells specific for non-glycosylated COL2, the inventors opted for the development of a non-galactosylated vaccine. Additionally, the complexity of synthesizing glycosylated vaccines presents significant challenges for scalability and further development. The selected peptides are depicted in Fig. 1a.

To screen their functional effect, we used the delayed-type hypersensitivity (DTH) model mediated by IFNy-producing type 1 T helper (Th1) cells, a test predicting an arthritis protective effect. Mice were vaccinated using osmotic pumps utilizing various complexes comprising human MHCII (DRB1*04:01, named DR4) combined with either the unmodified COL2₂₅₉₋₂₇₃ peptide, or modified peptides (AL170, AL177, AL178 and AL179). A DR4 complex containing a mutated CLIP (class II-associated invariant chain peptide) served as the control. Among the peptides tested, AL179 exhibited the most efficient reduction in ear swelling compared to the control group (Fig. 1b).

AL179 bound well to MHCII and demonstrated enhanced activation of COL2-specific T cell hybridoma (3H8) (Fig. 1c and 1d). In parallel, docking analysis of DR4-COL2₂₅₉₋₂₇₃ and DR4-AL179 with the TCR revealed an increased number of hydrogen bonds reflecting a stronger binding affinity and stabilization of the complex formation between DR4-AL179 and TCR compared to DR4-COL2₂₅₉₋₂₇₃. Furthermore, recognizing the need for a more applicable administration route for human translation, we compared osmotic pump delivery with three subcutaneous (s.c) administrations every 4 hours (hrs). Our findings revealed no discernible differences in ear swelling or IL-17A secretion (Fig. 1e and 1f, respectively), prompting the selection of the latter route for subsequent experiments.

### Example 2: DR4-AL179 vaccine alleviates autoimmune arthritis.

Next, the inventors assessed the efficacy of the DR4-AL179 vaccine in ameliorating arthritis in the CIA model. Treatment with DR4-AL179 significantly reduced both the incidence and the severity of arthritis (Fig. 2a). In contrast, the native non-glycosylated DR4-COL2₂₅₉₋₂₇₃ vaccine did not affect arthritis (Fig. 2a), despite previously showing a reduction in ear swelling in the DTH model (Fig. 1b and 1e). The therapeutic effect of DR4-AL179 in the CIA model was further supported by paw histology (Fig. 2b). Additionally, serum cytokine analysis on day 20 post-immunization revealed a marked increase in the anti-inflammatory IL-10 cytokine and a reduction in the pro-inflammatory cytokines IFN-γ and IL-6 (Fig. 2c) after vaccination with DR4-AL179 complex. Notably, spleen analysis at the terminal point (day 80) showed a significant reduction in COL2-specific T cells in the DR4-AL179-vaccinated group (Fig. 2d).

Moreover, vaccination with DR4-AL179 resulted in a notable decrease in anti-COL2 IgG antibody levels in comparison to the control group vaccinated with DR4-CLIP (Fig. 2e). This finding was corroborated by a bead-based immunoassay that detected epitope-specific anti-COL2 peptide IgG antibodies, revealing both epitope spreading over time and a marked reduction in antibody levels in the DR4-AL179 vaccination group, particularly against most COL2 peptides at day 45 post-immunization. Remarkably, DR4-AL179 vaccination significantly reduced the arthritogenic U1 antibody levels at day 45 post-immunization (Fig. 2f). Finally, the inventors demonstrated that the suppressive effect of T cells from DR4-AL179 vaccinated mice could be transferred, protecting recipient mice from CIA development (Fig. 2g).

### Example 3: Vaccination with DR4-AL179 expands antigen-specific CD4+ tolerogenic T cells.

The inventors found that the administration of DR4-AL179 to Parker mice led to a reduction in the number of COL2-specific T cells (Fig. 3a). The COL2-specific T cells from DR4-AL179 vaccinated mice were investigated for phenotypes earlier selected to be of importance for Aq-COL2 vaccination. Clearly, the T cells from the DR4-AL179 vaccinated mice had an increased expression of cell markers associated with a tolerogenic phenotype as compared with the DR4-CLIP control group. We observed higher programmed death-1 (PD-1) expression after DR4-AL179 vaccination (Fig. 3b). Similarly, CD73, folate receptor 4 (FR4) (Fig. 3c), and V-type immunoglobulin domain-containing suppressor of T-cell (VISTA) (Fig. 3d) were upregulated after vaccination with DR4-AL179. It has been reported that, CD73highFR4high T cells have the capacity to differentiate into either Foxp3-expressing Tregs or IL-10-producing CD49+Lag3+ unconventional regulatory T cells (Tr1 cells), depending on the specific physiological context. However, in this sense, the inventors observed that vaccination with DR4-AL179 did not increase the Foxp3 conventional regulatory T cells (Fig. 3e) but rather increased the non-classical regulatory T cells, Tr1 population (Fig. 3f).

VISTA, a member of the B7 family of immune inhibitory molecules, is known to negatively regulate immune responses, maintain peripheral tolerance, and control autoimmunity. Our previous work in tolerogenic vaccination hypothesized that VISTA plays a crucial role in vaccination efficacy in A^{q} mice. Literature suggests a higher expression of VISTA on regulatory T cells. Similarly, we observed a higher VISTA expression on Foxp3 Tregs (Fig. 3g), and in Tr1 T cells (Fig. 3h), although no difference in Foxp3 expression after vaccination with DR4-AL179 was observed (Fig. 3e).

Although vaccination with DR4-COL2₂₅₉₋₂₇₃ did not significantly ameliorate autoimmune arthritis in the CIA model (Fig. 2a), it led to increased expression of PD-1 and VISTA on COL2-specific T cells, as well as a higher frequency of CD73highFR4high T cells. A similar effect was observed also after DR4-AL179 vaccination. However, the increment of VISTA levels was less pronounced with DR4-COL2₂₅₉₋₂₇₃ than with DR4-AL179, suggesting a role of VISTA in the vaccine's mechanism of action.

### Example 4: The role of VISTA in shaping vaccine efficacy

To further investigate the role of VISTA on vaccination efficacy, the inventors conducted an *ex vivo* T cell suppression assay. Ten days post-immunization, COL2-specific T cells were enriched from the spleens of vaccinated mice and co-cultured with purified, cell trace-labeled CD4+ T cells from Parker mice, which were activated with anti-CD3 and anti-CD28 antibodies. The CD4+ T cells from vaccinated mice were pretreated with either anti-VISTA, anti-PD-1, or isotype control antibodies. Blocking VISTA resulted in a reduced suppressive effect compared to the isotype control, whereas blocking PD-1 had no significant impact (Fig. 4a).

To confirm VISTA's pivotal role in vaccination efficacy, the inventors performed an adoptive transfer experiment. CD4+ T cells from vaccinated mice were pretreated with either anti-VISTA or isotype control antibodies and transferred into immunized recipient Parker mice. A control group received T cells from unvaccinated mice. VISTA expression levels in different groups were assessed by flow cytometry before transfer (Fig. 4b). Blocking VISTA impaired the T cells' ability to ameliorate autoimmune arthritis, whereas T cells treated with the isotype control effectively suppressed arthritis (Fig. 4c), aligning with the results from the ex vivo T cell suppression assay (Fig. 4a). Moreover, VISTA remained inhibited for 35 DPI in peripheral blood, while VISTA expression was upregulated in the DR4-AL179 + isotype group compared to the control group (Fig. 4d), as shown previously (Fig. 3d). Histological analysis confirmed the severity of arthritis (Fig. 4e).

## Claims

1. **A modified COL2 peptide or protein,** wherein the modified COL2 peptide or protein comprises a modified COL2 amino acid sequence motif COL2₂₅₉₋₂₇₃; or a variant COL2₂₅₉₋₂₇₃ motif having at least 80% sequence identity to human COL2₂₅₉₋₂₇₃; wherein the modified COL2 amino acid sequence motif is **characterized by** a modification of the amino acid side chains at positions 263 and/or 265 of the motif compared to the (wild-type) human sequence motif COL2₂₅₉₋₂₇₃.

2. The modified COL2 peptide or protein of claim 1, wherein the modification is at position 263, wherein the modification is a chemical modification of the phenyl ring phenylalanine, such as a substitution of a H with OH, Met, MetO, or a halogen, such as F, Cl, Br, or I.

3. The modified COL2 peptide or protein of any one of claims 1 or 2, wherein the phenyl ring at position 263 is replaced with a pyridine, for example such that the nitrogen is placed in all five possible positions (excluding the connecting beta carbon of the amino acid).

4. The modified COL2 peptide or protein of any one of claims 1 to 3, wherein the PHE side chain of position 263 is modified by removing the -CH₂- group or extending it to -CH₂-CH₂-.

5. The modified COL2 peptide or protein of any one of claims 1 to 4, wherein the modified COL2 amino acid sequence motif comprises a sequence shown in SEQ ID NO 2 to 8, or 9 to 23.

6. The modified COL2 peptide or protein of any one of claims 1 to 5, which is not galactosylated at position Lysin₂₆₄.

7. The modified COL2 peptide or protein of any one of claims 1 to 6, wherein the modification is a replacement of the amino acid side chain at positions 263 and/or 265 with one of substituents selected from group consisting of:

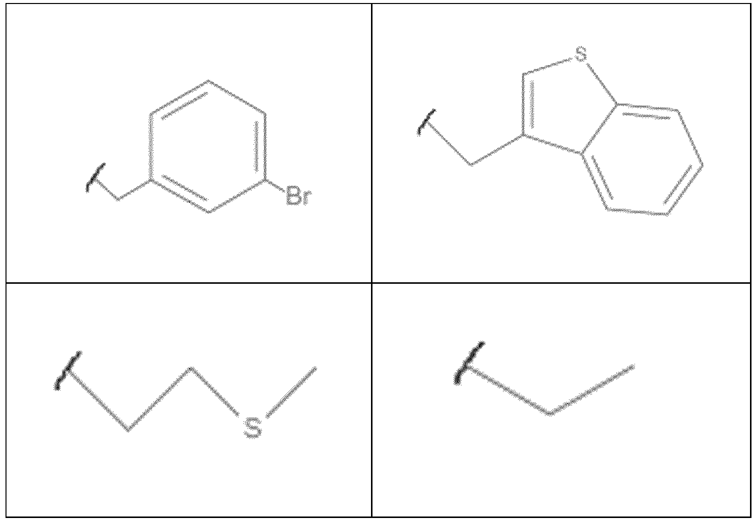

8. The modified COL2 peptide or protein of any one of claims 1 to 7, wherein the modification is a replacement of the amino acid side chain at position 263 with the chemical group:

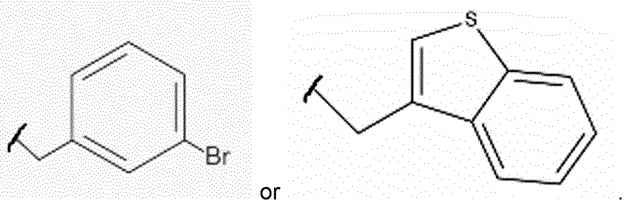

9. The modified COL2 peptide or protein of any one of claims 1 to 8, wherein the modification is a replacement of the amino acid side chain of position 265 with the group:

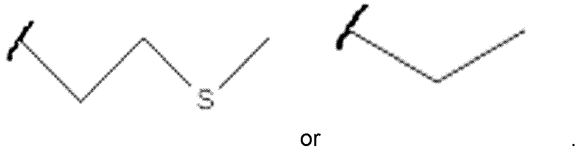

10. The modified COL2 peptide or protein of any one of claims 1 to 9, comprising one or more of the following modifications:
(i) On position 264: hydroxylation, hydroxy-galactosylation, or hydroxy-galactoglucosylation;
(ii) On position 270: hydroxylation, hydroxy-galactosylation, or hydroxy-galactoglucosylation;
(iii) On position 266: substitution to GLN (via amidation of GLU);
(iv) On position 272: substitution to GLN (via amidation of GLU);
(v) On position 269: substitution to GLU (via de-amidation of GLN);

11. **A protein complex** comprising
(i) A modified COL2 peptide or protein of any one of claims 1 to 10,
(ii) an extracellular region of an HLA-DR alpha chain comprising at least an alpha 1 domain;
(iii) an extracellular region of an HLA-DR beta chain comprising at least a beta 1 domain; and
wherein the modified COL2 is optionally fused to the N-terminus of the HLA-DR alpha chain or the HLA-DR beta chain by a linker peptide, preferably to the HLA-DR beta chain.

12. **A pharmaceutical composition,** comprising a modified COL2 peptide or protein of any one of claims 1 to 10, or a protein complex of claim 11, optionally together with a pharmaceutically acceptable carrier and/or excipient.

13. **A compound or composition for use in the treatment of a disease** in a subject, wherein the compound or composition is selected from, or comprises:
(i) a modified COL2 peptide or protein of any one of claims 1 to 10; and/or
(ii) a protein complex of claim 11; and/or
(iii) A pharmaceutical composition of claim 12.

14. The compound or composition for use of claim 13, wherein the disease is a chronic inflammatory disease, such as rheumatoid arthritis.

15. **A method for producing a composition,** the method comprising steps of
(i) Providing a modified COL2 peptide or protein of any one of claims 1 to 10 or a protein complex of claim 11;
(ii) admixing the modified COL2 peptide or protein or protein complex with at least one carrier and/or excipient to obtain the composition.
